# EUROPEAN PATENT SPECIFICATION

(11) **EP 1 175 230 B1**
(45) Date of publication and mention of the grant of the patent: **17.01.2007**
(21) Application number: 00901431.7
(22) Date of filing: 24.01.2000
(51) Int. Cl.: A61L 2/20, A61L 2/26

(54) **METHOD AND APPARATUS FOR OZONE STERILIZATION**
VERFAHREN UND APPARAT FÜR OZONE STERILISIERUNG
PROCEDE ET DISPOSITIF DE STERILISATION A L'OZONE

(30) Priority: 30.04.1999 CA 2270512
(43) Date of publication of application: 30.01.2002
(73) Proprietor: TS03 Inc., Saint-Foy, Quebec G1P 3S5 (CA)
(72) Inventor: SIMARD, Mario, Charlesbourg, Québec G1H 6Y1 (CA); FOURNIER, Stéphane, Levis, Québec G6V 7S9 (CA); DUFRESNE, Sylvie, Chalesbourg, Québec G1G 4W3 (CA); TURCOT, Richard, Cap-Rouge, Québec G1Y 2V5 (CA); ROBITAILLE, Simon, Charny, Québec G6X 3L1 (CA)
(74) Representative: Rapp, Bertram
(86) International application number: PCT/CA2000/000059
(87) International publication number: WO 2000/066186

(56) References cited:
- EP-A- 0 761 237
- WO-A-99/39723
- DE-A- 3 138 215
- FR-A- 2 759 590
- DATABASE WPI Section Ch, Week 199530 Derwent Publications Ltd., London, GB; Class D22, AN 1995-227446 XP002136448 & JP 07 136236 A (IWATATE IRYOUKIKI SEISAKUSHO YG), 30 May 1995 (1995-05-30)

## Description

### Field Of The Invention

The invention relates to sterilization equipment and, particularly, to a method and apparatus for ozone sterilization.

### Background Of The Invention

Sterilization is the absolute destruction of any virus, bacteria, fungus or other microorganism, whether in a vegetative or in a dormant spore state. Conventional sterile processing procedures for medical instruments involve high temperature (such as steam and dry heat units) or toxic chemicals (such as ethylene oxide gas, EtO). Steam pressure sterilization has been the time-honoured method of sterilization. It is fast and cost effective. However, the autoclave destroys heat-sensitive instruments. Thus, since more and more heat-sensitive instruments such as arthroscopes and endoscopes are used in medical treatment, other types of sterilization need to be used.

Ethylene oxide sterilization is used to cold sterilize heat-sensitive instruments. Until recently, ethylene oxide sterilization was the state of the art method for cold sterilization. Ethylene oxide sterilizes heat and moisture-sensitive objects and penetrates very well. However, it has been deemed by national health and safety organizations to be carcinogenic and neurotoxic. Additionally, since it is a highly flammable gas, it is normally combined with CFCs (chlorofluorocarbons) for safety reasons. However, due to the deleterious effects of CFCs on the ozone layer, their use has been banned by the Montreal protocol in 1996. Moreover, ethylene oxide requires long sterilization and aeration periods, since the molecule clings to the surface of instruments. The total sterilization time is 14 to 36 hours depending upon the materials to be sterilized. This type of sterilization necessitates the use of containment rooms, monitoring systems, and room ventilators.

A more efficient, safer, and less expensive sterilization agent was needed and has been found in the form of ozone O₃ which is the fourth most powerful, but overall most desirable oxidising agent (the three more powerful agents being fluorine derivatives which are too unstable and toxic for safe use in sterilization). Ozone can easily be generated from oxygen, especially hospital grade oxygen. Oxygen is readily available in the hospital environment, usually from a wall or ceiling oxygen source, or, if mobility is required, from a portable "J" cylinder of oxygen.

Ozone is widely used in industry as oxidising agent to bleach paper pulp, treat drinking water, and sterilize sewage water and food products. Ozone generally acts on chemical compounds in two ways. Either by direct reaction or through hydroxyl radical species formed during the decomposition of ozone (Encyclopaedia Of Chemical Technology, Vol. 17, Ozone page 953 to 964). The amounts (concentrations) of ozone required in the sterilization gas for water purification are low, generally less than 36 mg /l (milligram per litre). However, significantly higher concentrations are required to make ozone gas an effective sterilant of micro-organisms, those high concentrations of ozone gas have to be combined with critical levels of humidity during the entire sterilization cycle. The activity of ozone increases rapidly with increased relative humidity. The resistance of spores to ozone varies from strain to strain, but the differences become comparatively small at high relative humidity (Ishizaki et al., 1986. Inactivation of the Silas spores by gaseous ozone, J. Appl. Bacterial, 60:67-72), a high relative humidity is required for the ozone to penetrate the protective shells of micro-organisms. The presence of water often accelerates ozone reactions with organic substances (Langlais et al., (EDS), 1991, Ozone in Water Treatment, Application and Engineering. Louis Publishers: Chelsea, Michigan, 569 pages). Sufficient relative humidity is also required in order to enable ozone to penetrate the normally used sterilization packaging. Thus, it is desirable to humidify this ozone gas used for sterilization applications.

Various ways of humidifying ozone-containing gas used for sterilization treatments are known in the field of ozone sterilizers.

The use of a mixture of ozone gas with a very fine water mist in a sealed plastic bag container which contains an article to be sterilized is described in U.S. Patent No. 3,719,017. The method disclosed involves repeated evacuation and refilling of the plastic bag with a mixture of ozone gas and a very fine water mist. The air in the bag is exhausted and replaced with a pressurised mixture of ozone and water mist. Upon encountering the much lower pressure within the bag, the water particles from the pressurised mixture explode, forming a water mist. However, this system cannot generate a sufficiently high water vapour concentration to provide and maintain the required high relative humidity.

A review of more recent patents shows that the relative humidity required for successful sterilization is at least 85% throughout the process. U.S. Patent No. 5,069,880 describes a device capable of generating such a high relative humidity. In the apparatus described, the ozone gas is bubbled through a water bath in an effort to increase the water content of the gas. Although ozone at 85% humidity can kill most micro-organisms, it does not meet the "worst case scenario" stipulated in North American standards. Moreover, the device described is unable to generate humidity levels higher than 85%.

North American standards set by agencies such as the Food and Drug Administration and Heath Canada require sterilizer manufacturers to meet worst case scenario requirements. A sterilization gas including 85% humidity is insufficient for achieving the targeted results. A minimum relative humidity level of 95% is required to meet the standards imposed.

Water evaporates at 100C at atmospheric pressure (1013 mbar). Thus, various prior patents (see Faddis et al., U.S. Patents No. 5,266,275; 5,334,355; and 5,334,622) teach sterilization systems wherein water is heated to above the boiling point to evaporate the water for injection into the ozone-containing gas produced by an ozone generator. The steam is heated to 120C. Thus, the vapour upon injection into the ozone-containing gas presumably has a temperature close to 100C. However, since the decomposition of ozone increases exponentially with temperature in the range of 20 to 300C, injecting the water vapour at a temperature of about 120C leads to premature ozone decomposition. As a result, the effective ozone concentration in the gas produced by the ozone generator is reduced, thereby requiring significantly increased treatment times and the generation of larger amounts of ozone gas for each sterilization cycle. Thus, a more efficient and effective sterilization apparatus is desired for the sterilization of ozone at a relative humidity above of at least 95%.

French Patent FR 2759590 discloses a sterilization method including at least a cycle of steps which includes the steps of placing an article to be sterilized into a treatment chamber, reducing the pressure in the chamber to a first threshold pressure, injecting into the chamber a humidified ozone containing gas, carrying out an impregnation step at a pressure higher than the first threshold pressure, reducing the pressure until a second threshold pressure is reached and carrying out a sterilization step in a plasma gas atmosphere. The ozone containing gas is humidified by bubbling ozone containing gas through a aqueous solution, which is heated to about 80°C to elevate the relative humidity levels achieved. The resulting humidified gas is supplied to the sterilization chamber, which progressively raises the temperature in the chamber and increases the pressure therein. To avoid condensation of the water in the humidified gas at the increased pressure levels, a heating arrangement is provided to keep the temperature in the chamber above the due point.

Japanese Abstract JP-7-136236 teaches an ozone mixed steam sterilizing method. Steam and ozone are mixed prior to introduction into a sterilization chamber at negative pressure. No other method of humidification is disclosed. It is stated that the sterilization is carried out at increased temperature and humidity.

### Summary Of The Invention

It is an object of the invention to provide a method and apparatus for the sterilization of an article with ozone-containing gas, wherein the ozone-containing gas has a relative humidity above 95%, preferably at the saturation point, and a temperature at or close to the ambient temperature.

It is another object of the invention to provide a sterilization apparatus for ozone sterilization wherein the sterilization is carried out with humidified ozone-containing gas having a temperature of 20 to 30C.

It is still another object of the invention to provide an ozone sterilization apparatus wherein the sterilization is carried out at a temperature substantially equal to ambient temperature for allowing removal of the sterilized articles immediately after completion of the sterilization cycle, thereby avoiding extended cool-down periods.

It is yet a further object of the invention to provide an ozone sterilization apparatus wherein the sterilization period is significantly reduced by using an ozone-containing sterilization gas having a relative humidity above 95%, preferably about 100%.

These objects are achieved with a method according to claim 1 and apparatus according to claim 5 in accordance with the invention wherein sterilization is carried out under vacuum, whereby the vacuum pressure is selected such that the boiling temperature of water in the sterilization chamber is below the temperature inside the sterilization chamber.

In a preferred embodiment of the method and apparatus in accordance with the invention, a vacuum pressure is applied to lower the boiling point of water below the temperature inside the chamber. The cycle begins by applying a vacuum pressure preferably between 0.1 and 10 mbar, most preferably between 0.5 and 2.0 mbar.

The preferred sterilization method in accordance with the invention for the sterilization of an article includes the steps described in claim 1.

One or more ventilating cycles can be added to the preferred method for removing the remaining ozone and humidity from the sterilization chamber.

Accordingly, a sterilization apparatus in accordance with the invention includes the features described in claim 5.

### Brief Description of the Drawings

The invention will be described in more detail in the following by way of example only and with reference to the attached drawings wherein
FIG. 1 shows a schematic illustration of an apparatus in accordance with the invention;
FIG. 2 is a cross-section through a preferred ozone generator used in an apparatus in accordance with the invention;
FIG. 3 is a flow diagram of a preferred method in accordance with the invention;

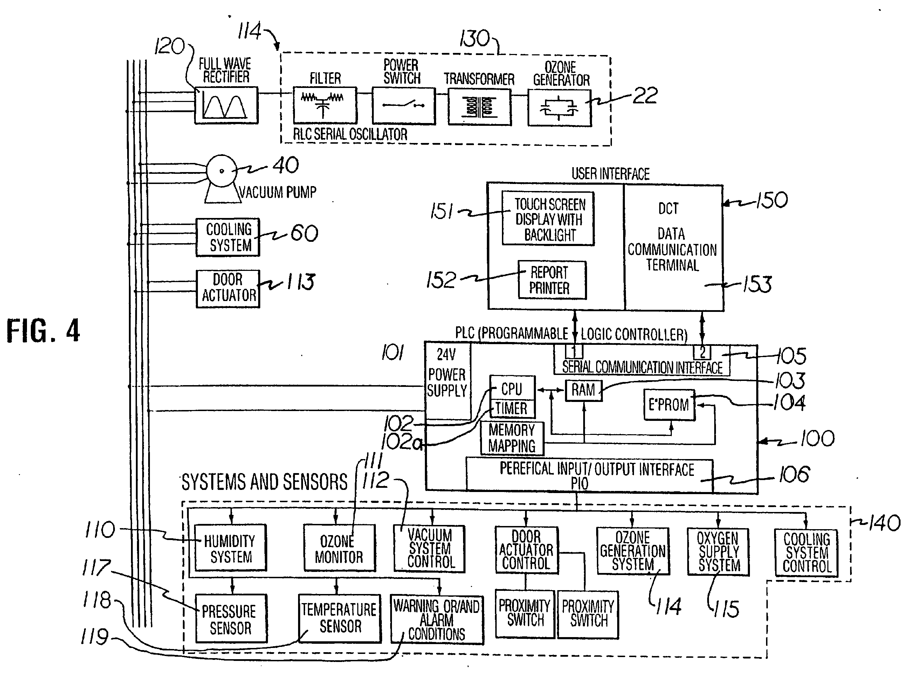

FIG. 4 is a flow diagram of the electrical and control system preferably used in the apparatus of FIG. 1; and
FIG. 5 is a schematic illustration of the cooling unit of the apparatus in accordance with the invention.

### Detailed Description of the Preferred Embodiment

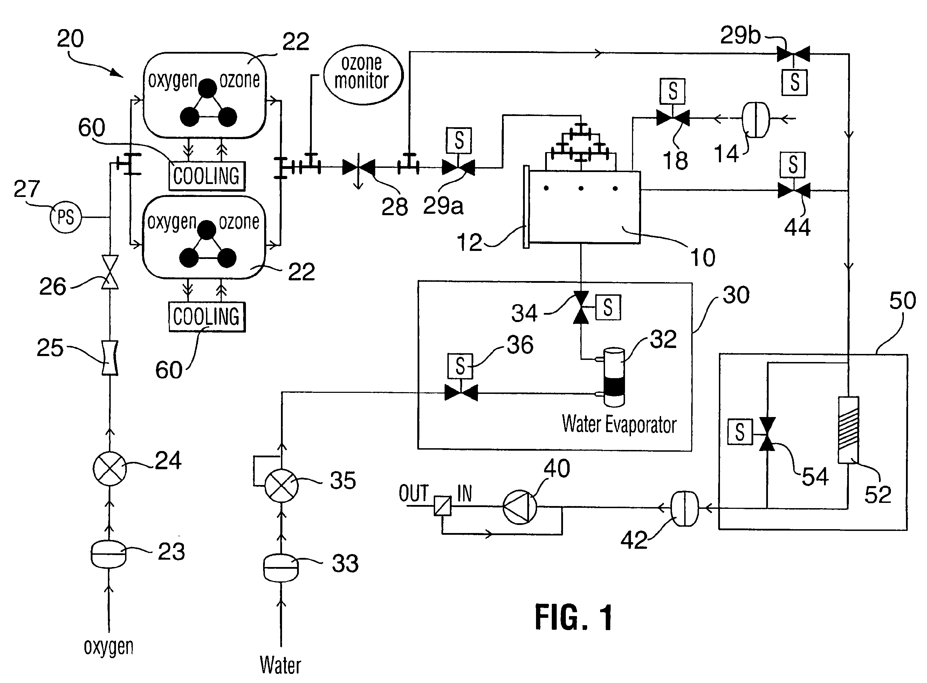

An ozone sterilizer in accordance with the invention as illustrated schematically in FIG. 1 operates in a relatively simple manner. Medical quality oxygen is subjected in an ozone-generating unit 20 to an electrical field, which converts the oxygen into ozone. The ozone is then fed into a humidified sterilization chamber 10 where it sterilises medical devices. The ozone is subsequently reconverted into oxygen using an ozone converting unit 50. The only residues left at the end of the sterilization cycle are oxygen and clean water vapour.

Single cycle sterilization with ozone is more efficient and provides for a shorter sterilization cycle than with ETO and requires few changes in user habits. Moreover, the ozone-based process in accordance with the invention is compatible for use with current packaging, such as sterile pouches and rigid containers.

The sterilization process of the invention is simple and substantially avoids human errors caused by false interpretation and handling.

The ozone sterilization method of the invention requires substantially no aeration or cooling down of sterilized instruments so that they can be used immediately following the sterilization cycle. This allows hospitals to reduce the cost of maintaining expensive medical device inventories. The ozone sterilization method of the invention offers several further advantages. It produces no toxic waste, does not require the handling of dangerous gas cylinders, and poses no threat to the environment or the user's health. Stainless-steel instruments and heat-sensitive instruments can be treated simultaneously, which for some users will obviate the need for two separate sterilizers.

The preferred sterilization apparatus in accordance with the invention as illustrated schematically in FIG. 1 includes a sterilization chamber 10 which can be sealed to contain a vacuum. This is achieved with an access door 12, which can be selectively opened for access into the chamber and which seals the chamber in the closed condition. The apparatus further includes an ozone generating unit 20 for supplying ozone-containing gas to the sterilization chamber, a humidifier arrangement 30 for supplying water vapour to the sterilization chamber, and a vacuum pump 40 (Trivac®, model D25BCS PFPE, manufacturer Leybold). The vacuum pump 40 is used for the application of a sufficient vacuum to the sterilization chamber 10 to increase the penetration of the sterilizing gas and to be able to generate water vapour at a temperature below the temperature inside the sterilization chamber. The vacuum pump 40 in the preferred embodiment is capable of producing a sufficient vacuum in the sterilization chamber to lower the boiling point of water in the chamber below the temperature in the chamber. In the preferred apparatus, the vacuum pump is capable of producing a vacuum of 0.1 mbar. Ozone produced in the ozone-generating unit 20 is destroyed in an ozone converting unit 50 to which ozone-containing gas is fed either after passage through the sterilization chamber 10 or directly from the ozone-generating unit 20 through valve 29b (optional). The ozone converting unit 50 includes an ozone converting catalyst 52 (DEST 25, manufacturer TSO3) and a by-pass valve 54 (optional). The ozone converting unit 50 is connected in series before or after the vacuum pump 40 to prevent ozone gas escaping to ambient. The ozone decomposing material in the preferred catalyst 52 is carulite. For economic and practical reasons, it is preferred to use a catalyst for decomposition of the ozone in the sterilization gas exhausted from the sterilization chamber 10. The catalyst destroys ozone on contact and retransforms it into oxygen with a certain amount of heat being produced. Catalysts of this type and their manufacture are well known to the person skilled in the art of ozone generators and need not be described in detail herein. Furthermore, other means for destroying the ozone contained in the sterilization gas will be readily apparent to a person skilled in the art. For example, the gas can be heated for a preselected time to a temperature at which the ozone decomposition is accelerated, for example, to 300C.

The humidifier arrangement 30 includes a humidifier chamber 32 (HUM 0.5, manufacturer TSO3) sealed to ambient and connected to the sterilization chamber 10 through a conduit and a vapour intake valve 34. The humidifier chamber 32 is equipped with a level control to always ensure a sufficiently high water level (not shown). Water is directly supplied to the humidifier chamber 32 from a drinking or purified water supply connection. Water is supplied to the humidifier chamber 32 by way of a filter 33, a pressure regulator 35, and input valve 36. The water vapour produced in the humidifier chamber 32 enters the sterilization chamber 10 by way of a vapour intake valve 34.

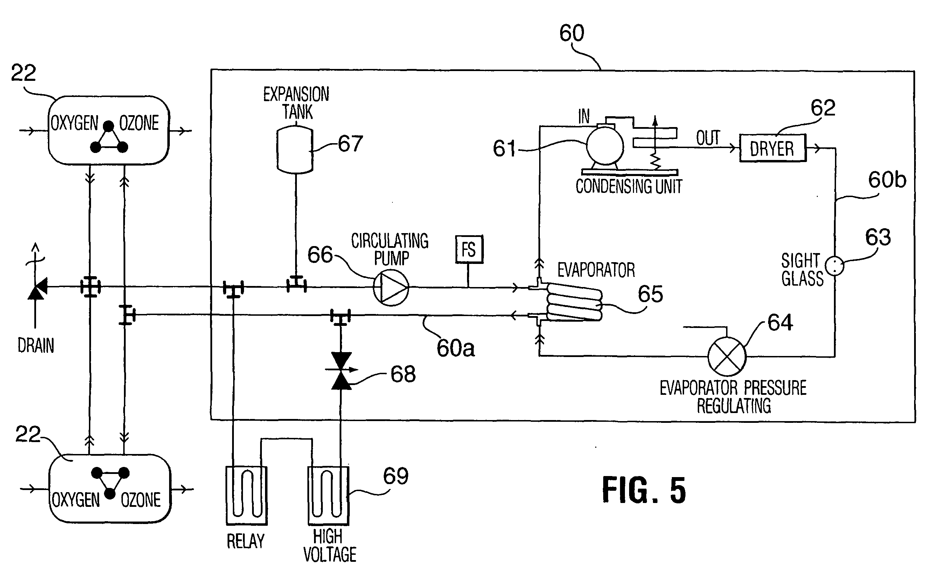

The ozone-generating unit 20 includes a pair ozone generators 22 (OZ, model 14a, manufacturer TS03) of the corona discharge type, which are cooled to decrease the ozone decomposition rate, which is well known in the art. To achieve a good lethality rate in an ozone sterilization process, the ozone supplied in the sterilization chamber should be sufficient to obtain a concentration of 48 to 96 milligram per litre preferably 60 to 72 milligram per litre. At these concentrations, the ozone generation is associated with a relatively high-energy loss in the form of heat. Generally, about 95% of the supplied electrical energy is converted into heat and only 5% is used to produce ozone. Since heat accelerates the inverse transformation of ozone into oxygen, it must be removed as quickly as possible by cooling the ozone generators 22. The ozone generators in the apparatus are kept at the relatively low temperature of 3 to 6C by either an indirect cooling system 60 as illustrated in FIG. 5 with cooling water recirculation, or a direct cooling system with a refrigeration unit for cooling (not illustrated). The cooling system is preferably kept at the temperature of 3 to 6C: In the preferred embodiment, the cooling system is kept at 4C so that the ozone-containing gas generated by unit 20 is at the ambient temperature around 20 to 35C. Thus, the ozone-containing gas entering into the sterilization chamber for humidification and sterilization is kept at ambient temperatures of 20 to 35C. This means that ozone decomposition is kept to a minimum and that the sterilization process is more efficient. This, provides a significant advantage over the apparatus of the prior art, since the temperature and pressure are maintained low throughout the sterilization cycle.

The ozone-generating unit is preferably supplied with medical quality oxygen. The apparatus can be connected to a wall oxygen outlet common in hospitals or to an oxygen cylinder or to any other source capable of supplying the required quality and flow. The supply of oxygen to the generators 22 takes place across a filter 23, a pressure regulator 24, a flow metre 25 and an oxygen shut off valve 26. The generators are protected against oxygen over pressure by a safety pressure switch 27. The ozone-oxygen mixture generated by the generators 22 is directed to the sterilization chamber 10 by a regulator valve 28 and a mixture supply solenoid valve 29a. The mixture can also be directly supplied to the ozone converting unit 52 by way of a bypass solenoid valve 29b (optional). In the preferred embodiment which includes a sterilization chamber of 125 liters volume, the pressure regulator 24 preferably controls the oxygen input at a flow rate of about 6 litres per minute. However, it will be readily apparent to the skilled person that other flow rates may be used depending on the make and model of the ozone generators 22 and the size of the sterilization chamber.

The apparatus in accordance with the invention preferably includes a closed circuit cooling system using absolutely no fresh water (see FIG. 5). The cooling liquid flowing inside the generators 22 is a glycol-water mixture which is cooled using R134a, an ozone layer friendly refrigerant. The cooling system is capable of maintaining a temperature of 3 to 6C and preferably 4C. The cooling system 60 of the generators 22 as shown in the schematic diagram of FIG. 5 includes a condensing unit 61 (Copelaweld FTAH-AO74, manufacturer: Copeland), a drier 62 (UK-O53S, manufacturer: Alco), a sight glass 63 (optional) (ALM-1 TT3, manufacturer: Alco), an expansion device 64 (Danfoss TEN2, manufacturer: Danfoss), an evaporator 65 (Packless COAX-2151-H, manufacturer: Packless), a circulation pump 66 well known to the person skilled in the art, and an expansion reservoir 67 (Amtrol ST-5, manufacturer: Amtrol). The cooling unit 60 is divided into a heat transfer circuit 60a and a refrigerating circuit 60b. The heat transfer circuit 60a includes the ozone generators 22, the high voltage circuit cooler 69, the coolant side of the evaporator 65, the circulation pump 66 and the expansion reservoir 67 (optional). The refrigeration circuit 60b includes the condensing unit 61, the drier 62, the sight glass 63, the expansion device 64 and the refrigerant side of the evaporator 65. The refrigerant circulating in the refrigeration circuit is R134a and the coolant flowing in the heat transfer circuit 60a is a glycol/water mixture.

The heat transfer circuit 60a can be omitted and the generators 22 included in the refrigeration circuit 60b. However, the use of an intermediate glycol/water filled heat transfer circuit is preferred, since the additional coolant acts as a larger heat sink so that energy peak loads generated upon activation of the generators 22 can be more reliably handled without significant swings in the temperature of the oxygen/ozone gas mixture produced.

The vacuum in the sterilization chamber 10 is produced by the vacuum pump 40 and across a filter 42, the ozone converting unit 52 and the sterilization chamber drainage valve 44.

Valves 18, 26, 29a, 29b, 34 and 36 are all the same (model: 0211-A-06,0-FF-VA-NM82-120/60-08, manufacturer: Burkert). Valves 44 and 54 are vacuum valves (model: DN 25 KF 287 66, manufacturer: Leybold).

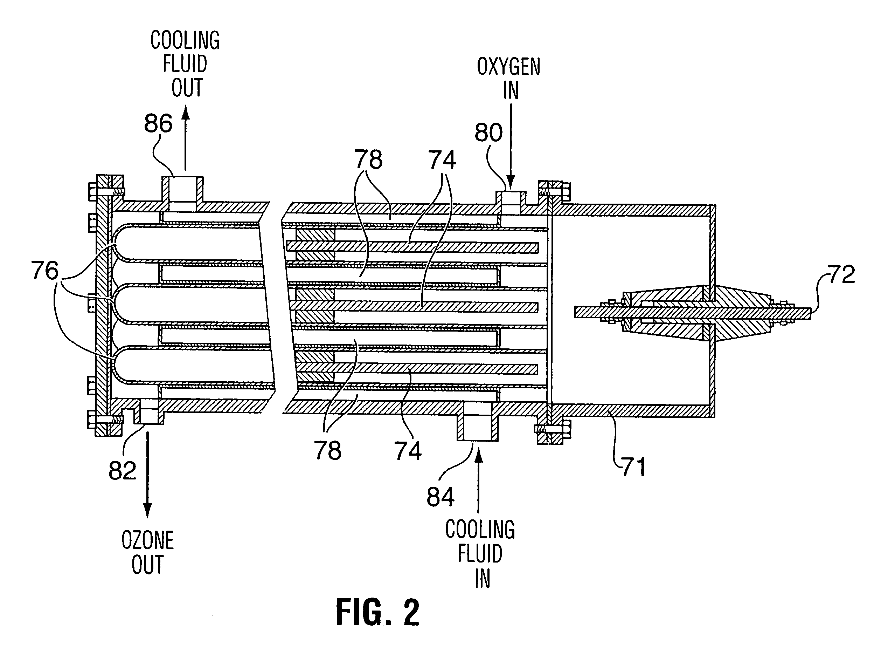

The preferred ozone generator used in the process and apparatus of the invention is schematically illustrated in Fig. 2 and is a generator of the corona discharge type well known to the person skilled in the art. The generator includes a first electrode 72 and a number of second electrodes 74 respectively centrally positioned in one of a corresponding number of reaction tubes 76. An ozone generating zone is defined between each second electrode 74 and the associated reaction tube 76. The electrodes are high voltage electrodes. Either electrode may be the ground electrode. The reaction tubes 76 are respectively surrounded by a cooling liquid channel 78 for cooling of the tubes. Oxygen enters the generator at an oxygen inlet 80 and ozone exits the generator at an ozone outlet 82. The reaction tubes are preferably made of a dielectric material, for example glas. The generator further includes an outer pressure vessel or housing 71 in which the oxygen inlet 80, ozone outlet 82 are provided as well as a cooling liquid inlet 84 and a cooling liquid outlet 86.

### Operation

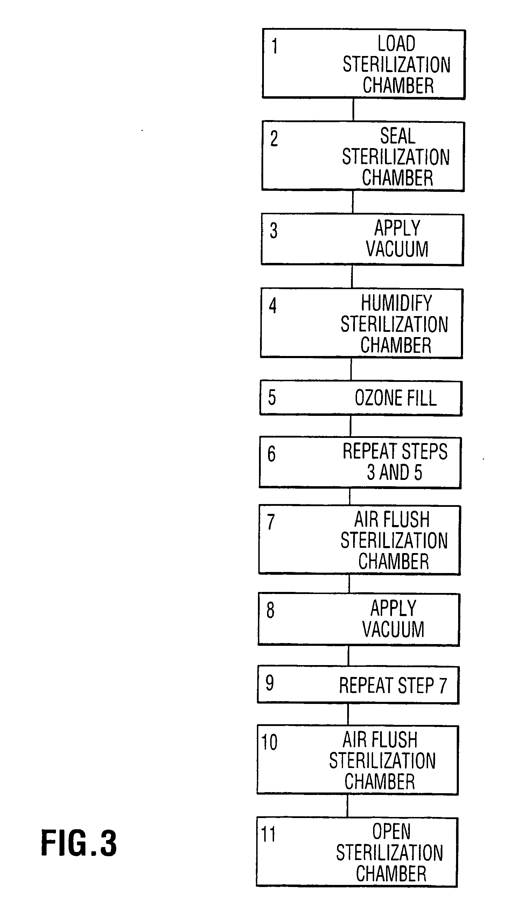

The preferred sterilization method according to the invention includes the following general steps as illustrated by the flow chart of FIG. 3. The medical instruments to be sterilized are sealed in sterile packaging containers or pouches such as generally used in the hospital environment and then placed into the sterilization chamber. The door of the sterilization chamber is closed and locked and the preconditioning phase is started by applying a vacuum to the sterilization chamber. Water vapour is admitted into the sterilization chamber to humidify the chamber contents. A mixture of ozone and oxygen is supplied to the chamber and the chamber maintained sealed for a preselected treatment period. Then the vacuum application and ozone supply steps are repeated at least once. To remove all remaining ozone in the sterilization chamber 10 when the sterilization cycle is completed a ventilation phase begins. After the ventilation phase the door is unlocked and the sterilized material can be taken out of the chamber.

Before the sterilization cycle begins, the humidifier chamber 32 is filled with water to an adequate level, which is sufficient to satisfy the requirements for the whole sterilization cycle. This is done by temporarily opening the water-input valve 36. Valve 36 remains closed for the whole remainder of the sterilization cycle. In the first phase of the sterilization cycle, air intake valve 18, oxygen shut-off valve 26, mixture supply valve 29a, and mixture bypass valve 29b are closed and vapour intake valve 34, chamber drainage valve 44, and bypass valve 54 are opened. The sterilization chamber 10 is evacuated to a vacuum pressure of about 0.1 mbar. Water vapour inlet valve 34 closes when the absolute pressure in the sterilization chamber falls below 60 mbar. Once a pressure of about 1,0 mbar is achieved, the chamber drainage valve 44 closes and the vapour intake valve 34 opens to lower the pressure in the humidifier chamber 32 to the vacuum pressure in the sterilization chamber. That forces the water in the humidifier chamber to evaporate and to enter the sterilization chamber 10. Shortly before the end of the humidification period (usually about 2 to 6 min.), the ozone generators are activated. The flow of the oxygen/ozone mixture exiting the ozone generator is controlled at all times by regulator valve 28 capable of resisting the vacuum and of adjusting the flow to between 4 and 12 litres per minute. As an optional feature, the generators can be started at the same time as the humidification period begins. This is then achieved with shut-off valve 26 and mixture bypass valve 29b. Shut-off valve 26 opens to let oxygen enter the generators. The ozone-oxygen mixture produced by the generators is then guided directly into the ozone converting unit 50 through mixture bypass valve 29b. After a humidification period of approximately 30 minutes, the oxygen-ozone mixture is guided into the sterilization chamber by opening the mixture supply valve 29a and closing the mixture bypass valve 29b. The oxygen-ozone mixture enters the chamber 10 until an ozone concentration of 72 milligram per litre in the chamber is achieved. The time required for this step is dependent on the flow rate and concentration of the ozone gas in the mixture (preferably 10 % to 12 % by weight). At this point in time, the mixture supply valve 29a is closed to seal off the sterilization chamber and to maintain the humidified ozone/oxygen gas mixture in the chamber under vacuum.

Once the sterilization chamber is filled with the sterilization gas (mixture of oxygen and ozone gas), the generators 22 are stopped, the oxygen shut-off valve 26 is closed, and the ozone is maintained in contact with the articles to be sterilized for about 20 minutes, for a sterilization chamber of a volume of 125 liters (4 cubic feet). The length of this sterilization period varies with the volume of the sterilization chamber. At this stage, the sterilization chamber is still under the effect of a partial vacuum of about 670 mbar. In an optional second step, the pressure level is raised to about 900 mbar using oxygen as a filling gas. This pressure level is maintained for about 20 min. After the sterilization period, the vacuum is reapplied, preferably at a pressure of about 1,0 mbar again. Once the vacuum reaches 0.1 mbar, the humidification phase is recommenced, followed by the renewed injection of an oxygen/ozone sterilization gas mixture, followed by the sterilization period. The cycle of applying a vacuum of about 1.0 mbar, injecting sterilization gas, humidifying and sterilization period, can be repeated, and the number of repeat cycles (mini cycles) selected to achieve complete sterilization of the instruments. The number of repeat cycles used in an experimental set-up of a method and apparatus in accordance with the invention including a 125 liters (4 cubic foot) chamber was 2 repeat cycles. This set-up conformed to the Security Assurance Level standards of the FDA (SAL 10 -6).

To remove all remaining ozone and humidity in the sterilization chamber 10 after complete sterilization a ventilation phase is engaged. The ventilation phase begins after the last sterilization period. The chamber drainage valve 44 opens and the vacuum is applied down to approximately 13 mbar. Vapour intake valve 34 closes when the pressure reaches 60 mbar to evacuate the remaining ozone in the humidifier. Once the vacuum pressure of 13 mbar is obtained, drainage valve 44 closes and the air intake valve 18 opens, admitting air into the sterilization chamber 10. Once atmospheric pressure is reached, the air intake valve 18 is closed, the sterilization chamber drainage valve 44 opened, and vacuum reapplied until a pressure of 13 mbar is reached. The ventilation cycle is then repeated twice. Once the atmospheric pressure is reached after the last cycle, the door mechanism of the sterilization chamber is activated to permit access to the contents of the sterilization chamber. This ventilation phase has two functions. First, to remove all ozone residues in the sterilization chamber before opening the access door and, second, to dry the sterilized material by evaporation when the vacuum pressure is applied.

The ozone-containing gas evacuated from the sterilization chamber 10 is passed over the ozone decomposing catalyst 52 of the ozone converting unit 50 prior to exhausting the gas to the atmosphere to ensure a complete decomposition of the ozone in the sterilization gas. The ozone converting unit 50 is used during only two portions of the sterilization cycle, the activation of the generators 22 (with optional valves 26 and 29b) and the evacuation of the sterilization chamber 10. During the start up phase of the generators 22, the mixture bypass valve 29b is opened and the ozone is guided across the catalyst 52. Once the start-up phase of the generators 22 is complete, the bypass valve 29b closes. During evacuation of the sterilization chamber 10, the sterilization chamber drainage valve 44 is opened and the ozone containing sterilization waste gas guided to the catalyst 52. Once the evacuation of the sterilization chamber 10 is completed, the drainage valve 44 is closed. The circulation of ozone is ensured by the vacuum pump 40, which operates during the whole sterilization cycle including all repeat cycles. If the ozone decomposing catalyst 52 is located upstream of the vacuum pump this also ensures that the carulite is kept as dry as possible in order to avoid fouling of the catalytic material. Since the vacuum pump 40 is running during the whole sterilization process, the carulite is kept in constant vacuum, even if it is not used for the decomposition of ozone. This forces of all water contained in the catalyst, which may have been absorbed by the carulite during the evacuation of the sterilization chamber. If located downstream of the vacuum pump, the catalyst will have to be heated to maintain the carulite sufficiently dry.

### Control System

The sterilization apparatus is preferably controlled by a programmable logic controller (PLC) 100. The control system includes a 24 volt power supply 101, a microprocessor 102, a random access memory (RAM) 103, and an erasable programmable memory (EPROM) 104, a communications interface 105 (Series RS-232), and an input output peripheral 106.

The input output peripheral 106 receives and sends information to and from the different systems and sensors 140 of the sterilization apparatus according to a specified sequence defined in the control protocol.

The humidification system 110 receives function commands from the PLC 100 according to the control protocol. The PLC 100 controls the water supply valve 36. A water level detector (not illustrated) allows the PLC 100 to close the water supply valve 36 once the desired water level is reached. An internal clock 102a of the PLC determines the humidification time within the sterilization chamber, which time is controlled for obtaining a relative humidity above 95%. The PLC 100 controls a temperature control to ensure a reliable functioning of the humidification system 110.

The ozone sensor 111 transmits two analog signals to the PLC 100 for determination of the ozone quantity produced by the generators 22. In the preferred embodiment, the ozone sensor is made up of a flow regulator, a carulite chamber and two temperature sensors 118. However, it will be readily apparent to a person skilled in the art that other ozone sensors can be used which provide an analog signal proportional to the ozone concentration detected.

The vacuum control system 112 includes the vacuum pump 40 and a pressure sensor 117. The start and stop operations of the vacuum pump are controlled according to the control protocol.

The sterilization chamber door actuator system 113 includes an electric drive of the screw type and two inductive sensors 113a, 113b which allow the detection of the presence of the door as part of the control protocol. The door opening system is also used in the alarm conditions management protocol 119 to assure the safety of the user.

The ozone generation system 114 includes a full wave rectifier 120, and an oscillator circuit (RLC) 130 including the two ozone generators 22. The RLC circuit 130 is mounted as a resonator using the non-ideal characteristics of the high voltage transformer 133, 120 v-0-120 v/4500 v. The PLC 100 controls the ozone production and ensures by way of the ozone sensor 111 that the concentration desired for sterilization is achieved and maintained throughout the sterilization cycle.

The oxygen supply system 115 includes the oxygen shut-off valve 26 and the gas sensor 21, as well as a 350 mbar (gauge) maximum gas pressure regulator 24. The sensors and regulators are an integral part of the alarm condition protocol to ensure the protection of the user.

The cooling system 60 is controlled independently of the sterilization cycle by a startup and stop protocol of the compressor and the glycol circulation pump.

The control system is provided with a user interface 150. In the preferred embodiment, this interface includes a touch-sensitive liquid crystal display (LCD) screen 151, a printer 152 for performance reports and a communications port 153 (Series RS-232) allowing the user to receive and transmit information necessary for use of the apparatus. It will be readily apparent to the person skilled in the art that other types of user interfaces can be used such as touch-sensitive pads, keyboards, or the like, and other types of communications interfaces.

The system in accordance with the invention is capable of maintaining a relative humidity level of 95 % or higher throughout the sterilization cycle.

The energy needed to evaporate the water during the humidification phase is taken from many sources. It is taken from the structure of the humidifier unit and the sterilization chamber and from the material to be sterilized. This contributes to a further cooling of the chamber, and its contents. In effect, at 20C, water boils up to an absolute pressure of 23.3 mbar and at 35C, water boils up to an absolute pressure of 56.3 mbar. The vacuum in the sterilization chamber is preferably adjusted at a pressure where the boiling temperature of water is lowered below the temperature in the sterilization chamber. That boiling temperature may be so low that, depending on the energy available from the surrounding structure and gases, the water in the humidifier chamber will freeze before it gets vaporized. The humidifier may also be cooled by the evaporation process to a point where condensation freezes to the external surface of the humidifier. This can be avoided in another preferred embodiment by heating the external surface of the humidifier sufficiently to keep the exterior of the humidifier unit and the water inside the humidifier chamber at room temperature. This is achieved with a heating arrangement (not illustrated) which will be readily apparent to the person of skill in the art.

The water vapour generated in the humidifier unit increases the relative humidity in the sterilization chamber. The humidification phase is continued until the relative humidity of the gas surrounding the medical instruments contained in the packaging pouches and containers reaches a minimum of 95%, preferably 100%. For a sterilization chamber of an approximate volume of 125 liters, the water vapour admission increases the pressure to about 53 mbar in the sterilization chamber.

Oxygen/ozone-containing sterilization gas is injected into the humidified sterilization chamber at ambient temperature. The ozone-containing gas is not heated as in the prior art. For optimum operation of a sterilizer in accordance with the invention and having a 125 liters chamber, a system is preferably used which is capable of generating an ozone flow of about 6 litres per minute containing about 72 mg/l of ozone to obtain at least at total of 9000 mg of ozone for each of the fillings of the sterilization chamber.

In another process not falling under the scope of the claims humidification of the sterilization chamber is carried out by a pair of atomizers. The water is supplied to each of the atomizers from a water tank hooked up to the drinking water supply or a purified water supply. Ozone is supplied to the atomizers from an ozone accumulation tank. The atomizers are made of ozone oxidation resistant material, and are installed directly in the sterilization chamber. When the vacuum level is reached in the sterilization chamber, the atomizers release water and ozone. The ozone is moistened inside the atomizer. The ozone/atomized water mixture penetrates the sterilization chamber. Injecting the water into the sterilization chamber under vacuum has the immediate effect of evaporating the water. The sterilization chamber operating temperature is 20 to 35C, a temperature at which water evaporates at pressures of 23.3 to 56.3 mbar. Thus, the water becomes vapour due to the vacuum created by the vacuum pump. The resulting ozone/water vapour mixture penetrates the material to be sterilized.

Changes and modifications in the specifically described embodiments can be carried out without departing from the scope of the invention which is intended to be limited only by the scope of the appended claims.

## Claims

1. A method for the sterilization of an article including the steps of
a) providing a sterilization chamber (10),
b) placing the article into the sterilization chamber (10),
c) sealing the sterilization chamber (10),
d) applying a vacuum to the sterilization chamber (10),
e) humidifying the atmosphere in the sterilization chamber (10),
f) supplying ozone containing gas to the sterilization chamber (10),
g) maintaining the sterilization chamber sealed for a sterilization treatment period, and
h) releasing the vacuum after the treatment period,
**characterized in that** the vacuum is applied prior to humidification of the sterilization chamber atmosphere, that the ozone is supplied after the humidification step, that the operating temperature in the sterilization chamber is maintained at a temperature of 20-35°C, preferably 20-30°C, that the humidification step e) is achieved by exposing an amount of water to the vacuum pressure in the sterilization chamber to evaporate the water, which amount of water is sufficient to achieve a relative humidity in the chamber of 95-100%, and by adjusting the vacuum pressure in the sterilization chamber (10) applied in step d) to a level sufficient to lower the boiling point of water to a temperature at least as low as the temperature in the sterilization chamber.

2. The method of claim 1, **characterized in that** the pressure in the sterilization chamber (10) is maintained during the humidification step e) between 0.1 and 10 mbar, most preferably between 0.5 and 2 mbar.

3. The method of claim 1, **characterized in that** the steps of applying the vacuum, humidifying the sterilization chamber and supplying ozone to the sterilization chamber are repeated at least once.

4. The method of claim 1 or 2, **characterized in that** during the step of releasing the vacuum, all gases evacuated from the sterilization chamber (10) are passed through an ozone converting unit (50) to prevent emission of ozone to the atmosphere.

5. A sterilization apparatus including
- a vacuum sterilization chamber (10),
- an ozone generating unit (20) for supplying ozone containing gas to the sterilization chamber (10),
- a regulator valve (28) and a mixture supply valve (29a) for controlling the supply of ozone containing gas to the sterilization chamber (10),
- a humidifier arrangement (30) for supplying an amount of water to the sterilization chamber,
- a vacuum pump (40) for applying a vacuum in the sterilization chamber,
**characterized in that** the humidifier arrangement (30) includes a humidifier chamber (32) connected to a water supply connection with a water-input valve (36) and to the sterilization chamber (10) through a conduit and a vapor-intake valve (34), whereby the humidifier chamber (32) can be filled with water to an adequate level by opening the water-input valve (36) and the atmosphere in the sterilization chamber (10) can be humidified by evacuating it and by opening the vapor-intake valve (34), forcing the water in the humidifier chamber (32) to evaporate and to enter the sterilization chamber (10).

6. The apparatus of claim 5, **characterized in that** the apparatus further includes a control system (100) for operating the vacuum pump (40) until an operating pressure in the chamber (10) is reached which is sufficient to lower the boiling point of water to a temperature at least as low as the temperature in the sterilization chamber, for operating the humidifier arrangement (30) after the operating pressure is reached to achieve humidification of the atmosphere in the sterilization chamber (10) at a level of 95-100 % relative humidity, and for thereafter operating the mixture supply valve (29a) for supplying ozone containing gas to the sterilization chamber (10) so that the relative humidity in the sterilization chamber (10) is controlled separately from the ozone concentration.

7. The apparatus of claim 6, **characterized in that** the control system (100) operates the vacuum pump (40) to generate an operating pressure between 0.1 and 10 mbar, preferably 0.5 to 2 mbar.

8. The apparatus of one of claims 5 to 7, **characterized in that** it further includes an ozone converting unit (50) for destroying any ozone released from the sterilization chamber (10).

9. The apparatus of one of claims 5 to 8, **characterized in that** the ozone generating unit (20) includes a cooling system (60) for cooling the ozone containing gas generated to a temperature of about 20-35°C, preferably 20-30°C, prior to entry thereof into the sterilization chamber (10).

10. The apparatus of one of claims 6 to 9, **characterized in that** the control system (100) includes a pressure sensor (117) for determining the pressure in the sterilization chamber.

11. The apparatus of one of claims 8 to 10, **characterized in that** the ozone converting unit is positioned upstream of the vacuum pump to prevent ozone damage to the pump and preferably includes an ozone destroying catalyst (52).

## Patentansprüche

1. Verfahren für die Sterilisation eines Gegenstandes mit den Schritten
a) Vorsehen einer Sterilisationskammer (10),
b) Legen des Gegenstandes in die Sterilisationskammer (10),
c) Abdichten der Sterilisationskammer (10),
d) Aufbringen eines Vakuums auf die Sterilisationskammer (10),
e) Befeuchten der Atmosphäre in der Sterilisationskammer (10),
f) Liefern von Ozon enthaltendem Gas zur Sterilisationskammer (10),
g) Halten der Sterilisationskammer abgedichtet für einen Sterilisationsbehandlungszeitraum, und
h) Aufheben des Vakuums nach dem Behandlungszeitraum,
**dadurch gekennzeichnet, dass** das Vakuum vor der Befeuchtung der Sterilisationskammeratmosphäre aufgebracht wird, dass das Ozon nach dem Befeuchtungsschritt geliefert wird, dass die Betriebstemperatur in der Sterilisationskammer auf einer Temperatur von 20-35°C, vorzugsweise 20-30°C gehalten wird, dass der Befeuchtungsschritt e) erreicht wird, indem eine Menge von Wasser dem Vakuumdruck in der Sterilisationskammer ausgesetzt wird, um das Wasser zu verdampfen, wobei die Menge an Wasser ausreicht, um eine relative Feuchtigkeit in der Kammer von 95-100 % zu erreichen, und indem der Vakuumdruck in der Sterilisationskammer (10), der in Schritt d) aufgebracht wird, auf einen Pegel eingestellt wird, der ausreicht, um den Siedepunkt von Wasser auf eine Temperatur zu senken, die mindestens so niedrig wie die Temperatur in der Sterilisationskammer ist.

2. Verfahren nach Anspruch 1, **dadurch gekennzeichnet, dass** der Druck in der Sterilisationskammer (10) während des Befeuchtungsschritts e) zwischen 0,1 und 10 mbar, am meisten bevorzugt zwischen 0,5 und 2 mbar gehalten wird.

3. Verfahren nach Anspruch 1, **dadurch gekennzeichnet, dass** die Schritte des Aufbringens des Vakuums, des Befeuchtens der Sterilisationskammer und des Lieferns von Ozon in die Sterilisationskammer mindestens einmal wiederholt werden.

4. Verfahren nach Anspruch 1 oder 2, **dadurch gekennzeichnet, dass** während des Schritts des Aufhebens des Vakuums alle Gase, die aus der Sterilisationskammer (10) ausgepumpt werden, durch eine Ozonumwandlungseinheit (50) geleitet werden, um die Emission von Ozon in die Atmosphäre zu verhindern.

5. Sterilisationsvorrichtung mit
- einer Vakuumsterilisationskammer (10),
- einer Ozonerzeugungseinheit (20) zum Liefern von Ozon enthaltendem Gas zur Sterilisationskammer (10),
- einem Reglerventil (28) und einem Gemischzuführungsventil (29a) zum Regeln der Zuführung von Ozon enthaltendem Gas zur Sterilisationskammer (10),
- einer Befeuchteranordnung (30) zum Liefern einer Menge an Wasser zur Sterilisationskammer,
- einer Vakuumpumpe (40) zum Aufbringen eines Vakuums in der Sterilisationskammer, **dadurch gekennzeichnet, dass** die Befeuchteranordnung (30) eine Befeuchterkammer (32) umfasst, die mit einer Wasserversorgungsverbindung mit einem Wassereingangsventil (36) und mit der Sterilisationskammer (10) durch eine Leitung und ein Dampfeinlassventil (34) verbunden ist, wobei die Befeuchterkammer (32) mit Wasser auf einen angemessenen Pegel gefüllt werden kann, indem das Wassereingangsventil (36) geöffnet wird, und die Atmosphäre in der Sterilisationskammer (10) durch Entleeren derselben und durch Öffnen des Dampfeinlassventils (34) befeuchtet werden kann, was das Wasser in der Befeuchterkammer (32) dazu bringt, zu verdampfen und in die Sterilisationskammer (10) einzutreten.

6. Vorrichtung nach Anspruch 5, **dadurch gekennzeichnet, dass** die Vorrichtung ferner ein Steuersystem (100) zum Betreiben der Vakuumpumpe (40), bis ein Betriebsdruck in der Kammer (10) erreicht ist, der ausreicht, um den Siedepunkt von Wasser auf eine Temperatur zu senken, die zumindest so niedrig wie die Temperatur in der Sterilisationskammer ist, zum Betreiben der Befeuchteranordnung (30), nachdem der Betriebsdruck erreicht ist, um die Befeuchtung der Atmosphäre in der Sterilisationskammer (10) auf einem Pegel von 95-100 % relativer Feuchtigkeit zu erreichen, und zum Betätigen danach des Gemischzuführungsventils (29a) zum Zuführen von Ozon enthaltendem Gas zur Sterilisationskammer (10), so dass die relative Feuchtigkeit in der Sterilisationskammer (10) separat von der Ozonkonzentration gesteuert wird, umfasst.

7. Vorrichtung nach Anspruch 6, **dadurch gekennzeichnet, dass** das Steuersystem (100) die Vakuumpumpe (40) betreibt, um einen Betriebsdruck zwischen 0,1 und 10 mbar, vorzugsweise 0,5 bis 2 mbar zu erzeugen.

8. Vorrichtung nach einem der Ansprüche 5 bis 7, **dadurch gekennzeichnet, dass** sie ferner eine Ozonumwandlungseinheit (50) zum Zerstören von irgendwelchem Ozon, das aus der Sterilisationskammer (10) freigesetzt wird, umfasst.

9. Vorrichtung nach einem der Ansprüche 5 bis 8, **dadurch gekennzeichnet, dass** die Ozonerzeugungseinheit (20) ein Kühlsystem (60) zum Kühlen des erzeugten Ozon enthaltenden Gases auf eine Temperatur von etwa 20-35 °C, vorzugsweise 20-30°C vor dem Eintritt desselben in die Sterilisationskammer (10) umfasst.

10. Vorrichtung nach einem der Ansprüche 6 bis 9, **dadurch gekennzeichnet, dass** das Steuersystem (100) einen Drucksensor (117) zum Bestimmen des Drucks in der Sterilisationskammer umfasst.

11. Vorrichtung nach einem der Ansprüche 8 bis 10, **dadurch gekennzeichnet, dass** die Ozonumwandlungseinheit stromaufwärts der Vakuumpumpe angeordnet ist, um eine Ozonbeschädigung an der Pumpe zu verhindern, und vorzugsweise einen Ozonzerstörungskatalysator (52) umfasst.

## Revendications

1. Procédé de stérilisation d'un article comprenant les étapes consistant à :
a) mettre à disposition une chambre de stérilisation (10);
b) placer l'article dans la chambre de stérilisation (10) ;
c) fermer la chambre de stérilisation (10) de façon hermétique ;
d) créer le vide dans la chambre de stérilisation (10) ;
e) humidifier l'atmosphère dans la chambre de stérilisation (10) ;
f) amener à la chambre de stérilisation (10) un gaz contenant de l'ozone ;
g) maintenir la chambre de stérilisation hermétiquement close pendant une période de traitement de stérilisation ; et
h) évacuer le vide au terme de la période de traitement,
**caractérisé en ce que** le vide est créé ou appliqué avant d'humidifier l'atmosphère de la chambre de stérilisation, **en ce que** l'ozone est apporté après l'étape d'humidification, **en ce que** la température de service ou de fonctionnement dans la chambre de stérilisation est maintenue à une température comprise entre 20 et 35 °C, de préférence entre 20 et 30 °C, **en ce que** l'étape d'humidification e) est réalisée en exposant une quantité d'eau à la pression du vide dans la chambre de stérilisation pour évaporer l'eau, laquelle quantité d'eau suffit à créer une humidité relative dans la chambre comprise entre 95 et 100 %, et en ajustant la pression du vide dans la chambre de stérilisation (10) appliquée dans l'étape d) jusqu'à un niveau suffisant pour abaisser le point d'ébullition de l'eau à une température au moins aussi basse que la température dans la chambre de stérilisation.

2. Procédé selon la revendication 1, **caractérisé en ce que** la pression dans la chambre de stérilisation (10) est maintenue au cours de l'étape d'humidification e) entre 0,1 et 10 mbar, de façon la plus préférée, entre 0,5 et 2 mbar.

3. Procédé selon la revendication 1, **caractérisé en ce que** les étapes consistant à faire le vide, à humidifier la chambre de stérilisation et à alimenter en ozone la chambre de stérilisation sont répétées au moins une fois.

4. Procédé selon la revendication 1 ou 2, **caractérisé en ce qu'**au cours de l'étape d'évacuation du vide, tous les gaz évacués de la chambre de stérilisation (10) sont passés à travers une unité de transformation de l'ozone (50) pour empêcher l'émission d'ozone dans l'atmosphère.

5. Appareil de stérilisation, comprenant :
- une chambre de stérilisation sous vide (10),
- une unité de production d'ozone (20) pour alimenter la chambre de stérilisation (10) en gaz contenant de l'ozone,
- une soupape de régulation (28) et une soupape d'alimentation de mélange (29a) pour la commande d'alimentation du gaz contenant de l'ozone à la chambre de stérilisation (10),
- un ensemble humidificateur (30) pour amener une certaine quantité d'eau à la chambre de stérilisation,
- une pompe à vide (40) pour faire le vide dans la chambre de stérilisation,
**caractérisé en ce que** l'ensemble humidificateur (30) comprend une chambre d'humidification (32) reliée à un raccord d'alimentation en eau pourvu d'une soupape de prise d'eau (36) et à la chambre de stérilisation (10) via une conduite et une soupape d'admission de vapeur (34), la chambre d'humidification (32) pouvant être remplie d'eau jusqu'à un niveau approprié en ouvrant la soupape de prise d'eau (36) et l'atmosphère dans la chambre de stérilisation (10) pouvant être humidifiée en l'évacuant et en ouvrant la soupape d'admission de vapeur (34), en forçant l'eau de la chambre d'humidification (32) à s'évaporer et à pénétrer dans la chambre de stérilisation (10).

6. Appareil selon la revendication 5, **caractérisé en ce qu'**il comprend en outre un système de commande ou de contrôle (100) pour faire fonctionner la pompe à vide (40) jusqu'à obtention d'une pression de service ou de fonctionnement dans la chambre (10) suffisante pour abaisser le point d'ébullition de l'eau à une température au moins aussi basse que la température dans la chambre de stérilisation, pour faire fonctionner l'ensemble humidificateur (30) une fois atteinte la pression de service pour réaliser l'humidification de l'atmosphère dans la chambre de stérilisation (10) à un niveau d'humidité relative compris entre 95 et 100 %, et pour ensuite faire fonctionner la soupape d'alimentation de mélange (29a) afin d'amener le gaz contenant de l'ozone à la chambre de stérilisation (10), de telle sorte que l'humidité relative dans la chambre de stérilisation (10) soit commandée séparément de la concentration en ozone.

7. Appareil selon la revendication 6, **caractérisé en ce que** le système de commande (100) actionne la pompe à vide (40) de façon à générer une pression de service comprise entre 0,1 et 10 mbar, de préférence entre 0,5 et 2 mbar.

8. Appareil selon l'une des revendications 5 à 7, **caractérisé en ce qu'**il comprend en outre une unité de transformation de l'ozone (50) pour détruire tout ozone sorti de ou relâché par la chambre de stérilisation (10).

9. Appareil selon l'une des revendications 5 à 8, **caractérisé en ce que** l'unité de production d'ozone (20) comprend un système de refroidissement (60) pour refroidir le gaz contenant de l'ozone généré à une température d'environ 20 à 35 °C, de préférence 20 à 30 °C, avant qu'il ne pénètre dans la chambre de stérilisation (10).

10. Appareil selon l'une des revendications 6 à 9, **caractérisé en ce que** le système de commande (100) comprend un capteur de pression (117) pour déterminer la pression dans la chambre de stérilisation.

11. Appareil selon l'une des revendications 8 à 10, **caractérisé en ce que** l'unité de transformation de l'ozone est disposée en amont de la pompe à vide de façon à empêcher un endommagement de la pompe par l'ozone et **en ce qu'**elle comporte de préférence un catalyseur de destruction de l'ozone (52).
